# EUROPEAN PATENT APPLICATION

(11) **EP 4 763 116 A2**
(43) Date of publication of application: **24.06.2026**
(21) Application number: 25216112.0
(22) Date of filing: 17.11.2025
(51) Int. Cl.: A61B 34/00, A61B 34/30, A61B 1/00

(54) **METHOD FOR DISPLAYING FIELD OF VIEW AREA AND SURGICAL ROBOT SYSTEM**

(30) Priority: 27.11.2024 CN 202411722435
(71) Applicant: Shenzhen Mindray Bio-Medical Electronics Co., Ltd., Shenzhen, Guangdong 518057 (CN); Wuhan Mindray Scientific Co., Ltd., Wuhan City, Hubei Province 430206 (CN)
(72) Inventor: PING, Junyu, Shenzhen, 518057 (CN); YUAN, Xiaowen, Shenzhen, 518057 (CN); LI, Yang, Shenzhen, 518057 (CN)
(74) Representative: KIPA AB

(57) **Abstract**

Provided are a method for displaying a field of view area, and a surgical robot system. Obtain a first endoscopic image captured by an endoscope of a surgical robot system at a first position. Respectively display, on the first display and the second display, a first image with a first field of view area, based on the first endoscopic image. Control the endoscope to move to a second position and to capture a second endoscopic image, when an instrument replacement is detected. A field of view area of the second endoscopic image is larger than a field of view area of the first endoscopic image. Switch the second display to display a second image with a second field of view area larger than the first field of view area, based on the second endoscopic image, avoiding accidental contact between the instrument and human body, reducing a risk of accidental injury.

## Description

### TECHNICAL FIELD

The disclosure relates to the technical field of medical devices, and more particularly to a method for displaying a field of view area and a surgical robot system.

### BACKGROUND

During an operation of a surgical robot, if a doctor needs to replace a surgical instrument, an assistant needs to implement a replacement operation for said surgical instrument at one end of an imaging tower. After the surgical instrument is pulled out and replaced, the assistant inserts a replaced surgical instrument back into a human body based on clinical experience. An insertion position of the replaced surgical instrument relies entirely on blind guessing, which may lead to a risk of accidental injury to organs due to limited field of view area, while inserting the replaced surgical instrument, resulting in a higher risk of accidental injury and a poor user experience.

### SUMMARY

In view of this, embodiments of this disclosure provide a method for displaying a field of view area, and a surgical robot system to reduce a risk of accidental injury and improve a user experience when replacing an instrument.

To achieve above objective(s), embodiments of this disclosure provide following technical solutions.

According to a first aspect, an embodiment provides a method for displaying a field of view area of an endoscopic image for a surgical robot system, wherein the surgical robot system at least includes a first display arranged on a surgical console and a second display arranged on an external imaging tower;
wherein the method includes:
obtaining a first endoscopic image, which is captured by an endoscope of the surgical robot system at a first position;
respectively displaying, on the first display and the second display, a first image with a first field of view area, based on the first endoscopic image;
controlling the endoscope of the surgical robot system to move to a second position and to capture a second endoscopic image, when an instrument replacement is detected, wherein a field of view area of the second endoscopic image, which is captured by the endoscope of the surgical robot system at the second position, is larger than a field of view area of the first endoscopic image, which is captured by the endoscope of the surgical robot system at the first position; and
switching the second display to display a second image with a second field of view area, based on the second endoscopic image, wherein the second field of view area is larger than the first field of view area.

In an embodiment, the method further includes: maintaining the first display to display a third image with the first field of view area, based on the second endoscopic image, when the instrument replacement is detected, while controlling the endoscope of the surgical robot system to move to the second position and to capture the second endoscopic image.

In an embodiment, maintaining the first display to display a third image with the first field of view area, based on the second endoscopic image, includes: enlarging and cropping the second endoscopic image to obtain the third image with the first field of view area; and displaying, on the first display, the third image with the first field of view area.

In an embodiment, controlling the endoscope of the surgical robot system to move to a second position and to capture a second endoscopic image, when an instrument replacement is detected, includes: determining that the instrument replacement is detected, when an operation for characterizing the instrument replacement, which operation is triggered by a user, is detected; and controlling the endoscope of the surgical robot system to move to the second position and to capture the second endoscopic image.

In an embodiment, the method further includes: controlling the endoscope of the surgical robot system to move back to the first position and to recapture a first endoscopic image; when the instrument replacement is determined to be completed; and switching back to respectively display, on the first display and the second display, a first image with the first field of view area, based on said first endoscopic image.

In an embodiment, controlling the endoscope of the surgical robot system to move back to the first position and to recapture a first endoscopic image; when the instrument replacement is determined to be completed; includes: determining that the instrument replacement is completed, when an operation for characterizing that the instrument replacement is completed, which operation is triggered by a user, is detected; and controlling the endoscope of the surgical robot system to move back to the first position and to recapture the first endoscopic image.

In an embodiment, a content of the second image with the second field of view area includes a content of the first image with the first field of view area.

In an embodiment, the first field of view area is rectangular, and the second field of view area is circular.

According to a second aspect, an embodiment provides a method for displaying a field of view area of an endoscopic image, including:
obtaining a first endoscopic image, which is captured by an endoscope of a surgical robot system at a first position;
displaying a first image with a first field of view area, based on the first endoscopic image;
controlling the endoscope of the surgical robot system to move to a second position and to capture a second endoscopic image, when an instrument replacement is detected, wherein a field of view area of the second endoscopic image, which is captured by the endoscope of the surgical robot system at the second position, is larger than a field of view area of the first endoscopic image, which is captured by the endoscope of the surgical robot system at the first position; and
switching to display a second image with a second field of view area, based on the second endoscopic image, wherein the second field of view area is larger than the first field of view area.

In an embodiment, controlling the endoscope of the surgical robot system to move to a second position and to capture a second endoscopic image, when an instrument replacement is detected, includes: determining that the instrument replacement is detected, when an operation for characterizing the instrument replacement, which operation is triggered by a user, is detected; and controlling the endoscope of the surgical robot system to move to the second position and to capture the second endoscopic image.

In an embodiment, the method further includes: controlling the endoscope of the surgical robot system to move back to the first position and to recapture a first endoscopic image; when the instrument replacement is determined to be completed; and switching back to respectively display a first image with the first field of view area, based on said first endoscopic image.

In an embodiment, controlling the endoscope of the surgical robot system to move back to the first position and to recapture a first endoscopic image; when the instrument replacement is determined to be completed; includes: determining that the instrument replacement is completed, when an operation for characterizing that the instrument replacement is completed, which operation is triggered by a user, is detected; and controlling the endoscope of the surgical robot system to move back to the first position and to recapture the first endoscopic image.

In an embodiment, a content of the second image with the second field of view area includes a content of the first image with the first field of view area.

In an embodiment, the first field of view area is rectangular, and the second field of view area is circular.

In an embodiment, the surgical robot system includes a first display arranged on a surgical console and a second display arranged on an external imaging tower;
displaying a first image with a first field of view area, based on the first endoscopic image; controlling the endoscope of the surgical robot system to move to a second position and to capture a second endoscopic image, when an instrument replacement is detected; and switching to display a second image with a second field of view area, based on the second endoscopic image; include:
respectively displaying, on the first display and the second display, the first image with the first field of view area, based on the first endoscopic image; and
controlling the endoscope of the surgical robot system to move to the second position and to capture the second endoscopic image, when the instrument replacement is detected, and just switching the second display to display the second image with the second field of view area, based on the second endoscopic image.

In an embodiment, controlling the endoscope of the surgical robot system to move to a second position and to capture a second endoscopic image, when an instrument replacement is detected, includes: controlling the endoscope of the surgical robot system to move to the second position and to capture the second endoscopic image, when the instrument replacement is detected; and maintaining the first display to display a third image with the first field of view area, based on the second endoscopic image.

In an embodiment, maintaining the first display to display a third image with the first field of view area, based on the second endoscopic image, includes: enlarging and cropping the second endoscopic image to obtain the third image with the first field of view area; and displaying, on the first display, the third image with the first field of view area.

According to a third aspect, an embodiment provides a method for displaying a field of view area of an endoscopic image, including:
obtaining a first endoscopic image, which is captured by an endoscope at a first position;
displaying a first image with a first field of view area, based on the first endoscopic image;
controlling the endoscope to move to a second position and to capture a second endoscopic image, when a first designated event is detected, wherein a field of view area of the second endoscopic image, which is captured by the endoscope at the second position, is larger than a field of view area of the first endoscopic image, which is captured by the endoscope at the first position; and
switching to display a second image with a second field of view area, based on the second endoscopic image, wherein the second field of view area is larger than the first field of view area.

In an embodiment, controlling the endoscope to move to a second position, when a first designated event is detected, includes:
controlling the endoscope to move to the second position, when an instrument replacement is detected, or when an operation for turning on a function for switching a field of view area, which operation is triggered by a user, is detected.

In an embodiment, after switching to display a second image with a second field of view area, the method further includes:
controlling the endoscope to move back to the first position, and switching back to display the first image with the first field of view area, when a second designated event is detected.

In an embodiment, controlling the endoscope to move back to the first position, and switching back to display the first image with the first field of view area, when a second designated event is detected, include:
controlling the endoscope to move back to the first position, and switching back to display the first image with the first field of view area, when an instrument replacement is determined to be completed, or when an operation for turning off a function for switching a field of view area, which operation is triggered by a user, is detected.

According to a fourth aspect, an embodiment provides a method for displaying a field of view area of an endoscopic image for a surgical robot system, wherein the surgical robot system at least includes a first display arranged on a surgical console and a second display arranged on an external imaging tower;
the method includes:
obtaining a first endoscopic image, which is captured by an endoscope of the surgical robot system at a first position;
respectively displaying, on the first display and the second display, a first image with a first field of view area, based on the first endoscopic image;
implementing an assistance operation for an instrument replacement, when the instrument replacement is detected;
controlling the endoscope of the surgical robot system to move to a second position and to capture a second endoscopic image, when an operation for turning on a function for switching a field of view area, which operation is triggered by a user, is detected; wherein a field of view area of the second endoscopic image, which is captured by the endoscope of the surgical robot system at the second position, is larger than a field of view area of the first endoscopic image, which is captured by the endoscope of the surgical robot system at the first position; and
switching the second display to display a second image with a second field of view area, based on the second endoscopic image, wherein the second field of view area is larger than the first field of view area.

According to a fifth aspect, an embodiment provides a surgical robot system including a processor and a memory; wherein the memory includes computer-readable instruction(s) stored therein, wherein the computer-readable instruction(s), when executed by the processor, implements(implement) any one of the method for displaying a field of view area of an endoscopic image described above.

From above technical solutions, it can be seen that embodiments of this disclosure have following advantages.

When a surgical robot system is detected to replace an instrument, move an endoscope to obtain an endoscopic image with a larger field of view area, and switch to display an image with a larger field of view area based on the endoscopic image with a larger field of view area, which image is obtained by imaging after the movement, so as to assist a user to observe an end position of the instrument in real time, thus avoiding accidental contact between the instrument and human body, reducing a risk of accidental injury and improving a user experience.

### BRIEF DESCRIPTION OF THE DRAWINGS

In order to provide a clearer explanation for embodiments of this disclosure or technical solutions in the prior art, a brief introduction is given to accompanying drawings required for description of the embodiments or the prior art. It is obvious that the accompanying drawings described below are only embodiments of this disclosure. For those skilled in the art, other drawings can be obtained based on the provided drawings without creative labour.
FIG. 1 is a structure diagram for a surgical robot system in an embodiment of this disclosure.
FIG. 2 is a first flowchart for a method for displaying a field of view area of an endoscopic image for a surgical robot system in an embodiment of this disclosure.
FIG. 3 is an example diagram for a first image with a first field of view area in an embodiment of this disclosure.
FIG. 4 is another example diagram for a second image with a second field of view area in an embodiment of this disclosure.
FIG. 5 is an example display diagram for a second image with a second field of view area, which is captured at a second position in an embodiment of this disclosure.
FIG. 6 is another example display diagram for a second image with a second field of view area, which is captured at a second position in an embodiment of this disclosure.
FIG. 7 is an example workflow diagram for a method for displaying a field of view area of an endoscopic image in an embodiment of this disclosure.
FIG. 8 is an example diagram for switching a field of view area of a second display in an embodiment of this disclosure.
FIG. 9 is a second flowchart for a method for displaying a field of view area of an endoscopic image in an embodiment of this disclosure.
FIG. 10 is a third flowchart for a method for displaying a field of view area of an endoscopic image in an embodiment of this disclosure.
FIG. 11 is a fourth flowchart for a method for displaying a field of view area of an endoscopic image for a surgical robot system in an embodiment of this disclosure.
FIG. 12 is a fifth flowchart for a method for displaying a field of view area of an endoscopic image for a surgical robot system in an embodiment of this disclosure.
FIG. 13 is an example diagram for a photosensitive range, a first field of view area, and a second field of view area of an image sensor in an embodiment of this disclosure.
FIG. 14 is an example display diagram for a second image with a second field of view area in an embodiment of this disclosure.
FIG. 15 is another example display diagram for a second image with a second field of view area in an embodiment of this disclosure.
FIG. 16 is another example workflow diagram for a method for displaying a field of view area of an endoscopic image in an embodiment of this disclosure.
FIG. 17 is another example diagram for switching a field of view area of a second display in an embodiment of this disclosure.
FIG. 18 is a sixth flowchart for a method for displaying a field of view area of an endoscopic image in an embodiment of this disclosure.
FIG. 19 is a seventh flowchart for a method for displaying a field of view area of an endoscopic image in an embodiment of this disclosure.
FIG. 20 is an eighth flowchart for a method for displaying a field of view area of an endoscopic image for a surgical robot system in an embodiment of this disclosure.

### DESCRIPTION OF THE PREFERRED EMBODIMENTS

Technical solutions in embodiments of this disclosure will be clearly and completely described in conjunction with the accompanying drawings in the followings. Obviously, the described embodiments are only a part of the embodiments of this disclosure, not all of them. Based on the embodiments of this disclosure, all other embodiments obtained by ordinary skilled persons in the art without creative labor are within a scope of protection of this disclosure.

In this disclosure, the terms "including", " containing", or any other variation thereof are intended to encompass non-exclusive inclusion, such that a process, method, article, or device that includes a series of elements not only includes those elements, but further includes other elements that are not explicitly listed or are inherent to such a process, method, article, or device. Without further limitations, an element defined by the statement "including a..." does not exclude an existence of another identical element in the process, method, article, or device that includes the element.

During an operation of a surgical robot (such as a laparoscopic surgical robot), if a doctor needs to replace a surgical instrument, an assistant needs to implement a replacement operation of said surgical instrument at one end of an imaging tower. After a surgical instrument is disassembled and replaced, a replaced surgical instrument needs to be inserted back into a human body. Due to a limited range of traditional 3D imaging, the assistant will insert the replaced surgical instrument back into the human body based on clinical experience. An insertion position of the replaced surgical instrument relies entirely on blind guessing, which may lead to a risk of accidental injury to organs due to limited field of view area, while inserting the replaced surgical instrument.

The inventor found through researches that an existing surgical robot has a position memory function, and a surgical instrument can reach a peripheral position before the surgical instrument is pulled out, while being inserted for the second time. Although this leaves a corresponding space for the surgical instrument, however, a relative relationship between organs in the human body may change at any time. Accordingly, although a space is left for the second insertion of the surgical instrument, it may still accidentally injure the organs. Another existing surgical robot has a position memory function and a target position marking function. It will automatically stop when encountering obstacles in a path for a second insertion, but this cannot avoid a risk of accidental injury through vision.

Based on the above research content, this disclosure proposes a method for displaying a field of view area of an endoscopic image and a surgical robot system. When a surgical robot system is detected to replace an instrument, move an endoscope to capture an endoscopic image with a larger field of view area, and switch to display an image with a larger field of view area based on the endoscopic image with a larger field of view area, which image is captured after the movement, so as to assist a user to observe an end position of the instrument in real time, thus avoiding accidental contact between the instrument and human body, reducing a risk of accidental injury and improving a user experience.

By applying some embodiments of this disclosure, one practical application situation is to respectively display, on a first display and a second display, a first image with a first field of view area, based on a first endoscopic image which is captured by an endoscope of a surgical robot system at a first position; control the endoscope to move to a second position and to capture a second endoscopic image, when an instrument is replaced, wherein a field of view area of the second endoscopic image is larger than a field of view area of the first endoscopic image; switch the second display to display a second image with a second field of view area, based on the second endoscopic image, wherein the second field of view area is larger than the first field of view area; and use the second image with a larger field of view area to assist a user in observing an end position of the instrument in real time, thus avoiding accidental contact between the instrument and the human body.

Below, this disclosure will be described in detail through contents of various embodiments.

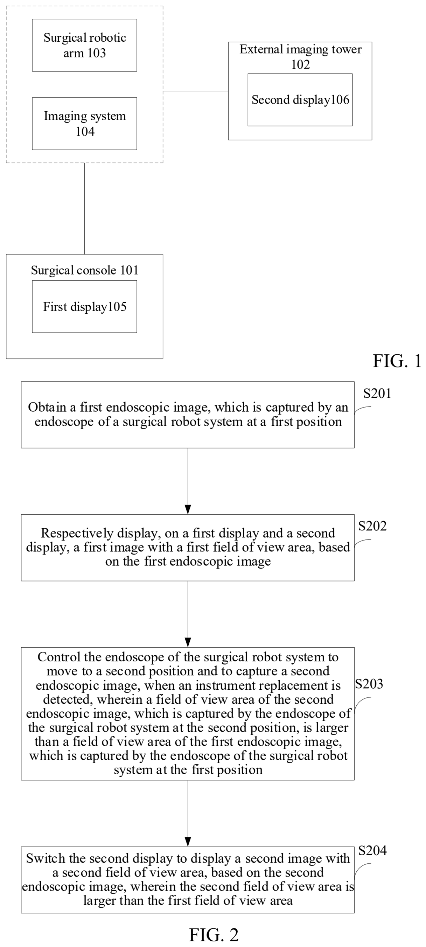

FIG. 1 is a structure diagram for a surgical robot system in an embodiment of this disclosure. The surgical robot system may include a surgical console 101, an external imaging tower 102 (also known as an imaging tower), a surgical robotic arm 103, an imaging system 104 (usually a 3D imaging system or 3D image system), a first display 105 arranged on the surgical console 101, and a second display 106 arranged on the external imaging tower 102. The imaging system 104 at least includes an endoscope, a processor and a memory.

Wherein, a chief surgeon of a surgical team controls the surgical robotic arm 103 to implement a surgery through the surgical console 101. The imaging system 104 collects image information during a surgical process and sends the collected image information to the first display 105 and the second display 106 for displaying.

The chief surgeon observes a surgical situation through the first display 105 of the surgical console 101, while other members of the surgical team (such as an assistant) observe the surgical situation through the second display 106 of the external imaging tower 102.

The memory in the imaging system 104 includes computer-readable instruction(s) stored therein, wherein the computer-readable instruction(s), when executed by the processor in the imaging system 104, implements(implement) any one of the method for displaying a field of view area of an endoscopic image described below.

It should be noted that, a structure of a surgical robot system shown in FIG. 1 is only illustrative and may include more or fewer components than shown in FIG. 1, or may have different configurations than shown in FIG. 1. The components shown in FIG. 1 can be implemented using hardware(s) and/or software(s).

Referring to FIG. 2, a first flowchart for a method for displaying a field of view area of an endoscopic image for a surgical robot system in an embodiment of this disclosure is shown. The surgical robot system at least includes a first display arranged on a surgical console and a second display arranged on an external imaging tower. FIG. 2 includes following steps.

In step S201, obtain a first endoscopic image, which is captured by an endoscope of the surgical robot system at a first position.

In a specific implementation of step S201, during a surgical process, a first endoscopic image, which is captured by the endoscope (e.g. stereo endoscope) of the surgical robot system (e.g. laparoscopic surgical robot system) at the first position, is obtained. The endoscope is arranged inside with an image sensor, and the first endoscopic image is specifically captured by the image sensor.

In step S202, respectively display, on the first display and the second display, a first image with a first field of view area, based on the first endoscopic image.

In a specific implementation of step S202, select the first image with the first field of view area from the captured first endoscopic image, and then respectively display, on the first display arranged on the surgical console and the second display arranged on the external imaging tower, the first image with the first field of view area.

It should be noted that, a shape of a conventional display is usually rectangular. In some specific embodiments, in order to further improve a user experience, the first field of view area can be rectangular (or other shapes), when displaying the first image with the first field of view area on the first display and the second display. This allows the first image with the first field of view area to fill a whole display screen, when displaying the first image with the first field of view area on the first display and the second display.

In step S203, control the endoscope of the surgical robot system to move to a second position and to capture a second endoscopic image, when an instrument replacement is detected, wherein a field of view area of the second endoscopic image, which is captured by the endoscope of the surgical robot system at the second position, is larger than a field of view area of the first endoscopic image, which is captured by the endoscope of the surgical robot system at the first position.

It should be noted that, a process for replacing an instrument in a surgical robot system is as follows. After determining that an instrument needs to be replaced, a user (such as an assistant) unlocks an instrument to be disassembled on the surgical robotic arm, disassembles said instrument, and assembles a new instrument to the surgical robotic arm, and locks the newly assembled instrument.

In a specific implementation of step S203, after displaying the first image with the first field of view area on the first display and the second display; when an instrument replacement is detected, control the endoscope of the surgical robot system to move from the first position to the second position, and control the endoscope to capture the second endoscopic image at the second position.

In some specific embodiments, when an operation for characterizing the instrument replacement, which operation is triggered by a user, is detected, determine that the instrument replacement is detected, and control the endoscope of the surgical robot system to move to the second position, and to capture the second endoscopic image.

For example, when an operation for unlocking an instrument, which operation is triggered by a user, is detected, determine that an instrument replacement is detected. At this time, control the endoscope of the surgical robot system to move to the second position, and to capture the second endoscopic image.

Specifically, when an instrument replacement is detected, control the endoscope of the surgical robot system to move from the first position to the second position along an axial direction toward an outside of a human body, that is, the endoscope moves from the first position to the second position along a center axis of the endoscope in a direction towards an outside of the human body, and to capture the second endoscopic image.

It should be noted that, imaging ranges of the endoscope vary at different positions, which results in different fields of view area for endoscopic images, which images are captured at different locations. Due to a movement of the endoscope towards the outside of the human body to the second position, an imaging range of the endoscope increases. Therefore, a field of view area of the second endoscopic image captured by the endoscope at the second position is larger than a field of view area of the first endoscopic image captured by the endoscope at the first position.

A distance between the first position and the second position can be pre-set, that is, the endoscope moves from the first position for a preset distance along a central axis of the endoscope in a direction towards the outside of the human body, so as to reach the second position. When the endoscope moves from the first position to the second position, in order to further ensure that a display content of the first display remains unchanged in subsequent steps, the endoscope can be controlled to rotate a preset angle, while moving from the first position to the second position.

In step S204, switch the second display to display a second image with a second field of view area, based on the second endoscopic image, wherein the second field of view area is larger than the first field of view area.

In a specific implementation of step S204, after controlling the endoscope to capture a second endoscopic image at the second position, select the second image with the second field of view area from the second endoscopic image, and control the second display to switch to display the second image with the second field of view area, so as to expand an observation field of view area.

It should be noted that, a field of view area of the second endoscopic image captured by the endoscope at the second position is larger than a field of view area of the first endoscopic image captured by the endoscope at the first position, so that the second field of view area is larger than the first field of view area.

In some specific embodiments, a content of the second image with the second field of view area includes a content of the first image with the first field of view area. Switching the second display from displaying the first image with the first field of view area to displaying the second image with the second field of view area can expand an observation field of view area displayed by the second display, thereby assisting a user in observing an end position of an instrument to avoid accidental injury.

In other embodiments, the first field of view area may be rectangular, and the second field of view area may be circular or rectangular. However, regardless of whether the second field of view area is circular or rectangular, the second field of view area is larger than the first field of view area. Therefore, switching the second display to display the second image with the second field of view area can expand an observation field of view area.

It should be noted that, an observation field of view area of a circular second field of view area is larger than an observation field of view area of a rectangular second field of view area. In practical applications, a shape of the second field of view area can be set according to an actual situation.

For example, if it is necessary to fill the entire second display with a second image with a second field of view area, then the second field of view area can be rectangular. If a larger observation field of view area is needed, the second field of view area can be circular.

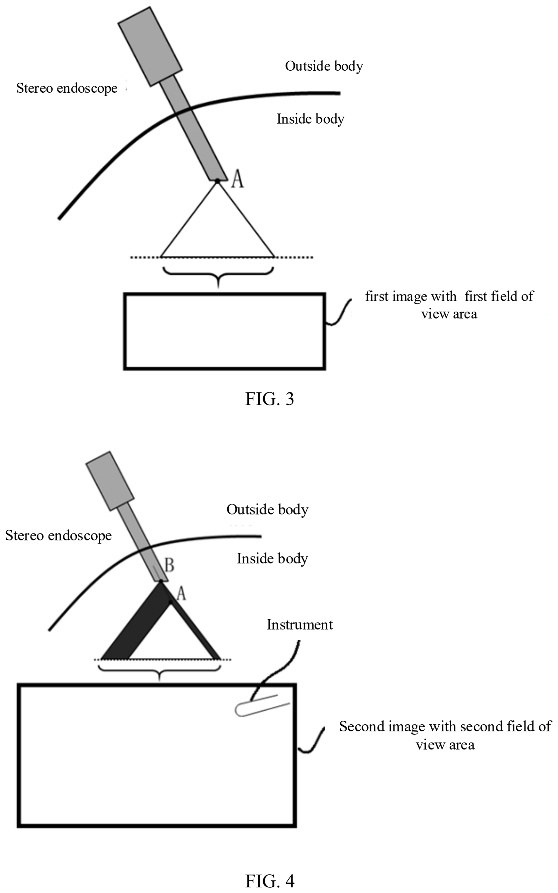

For example, assuming that both the first field of view area and the second field of view area are rectangular, a stereo endoscope captures a first endoscopic image at the first position. From the first endoscopic image, the first image with the first field of view area shown in FIG. 3 is selected, and the first image with the first field of view area is displayed on the first display and the second display, wherein point A in FIG. 3 is the first position.

When detecting an instrument replacement, control the stereoscopic endoscope to move from the first position to the second position along an axial direction in a direction toward an outside of a human body, and control the stereoscopic endoscope to capture a second endoscopic image, select the second image with the second field of view area as shown in FIG. 4 from the second endoscopic image, and switch the second display to display the second image with the second field of view area, wherein point A in FIG. 4 is the first position and point B in FIG. 4 is the second position.

From contents shown in FIGS. 3 and 4, it can be seen that switching the second display to display a second image with a second field of view area can expand an observation field of view area, thus allowing a user to observe an end position of the instrument in the second image with a larger field of view area.

It should be noted that, when the second field of view area is circular, there are two situations, in which the second display is switched to display the second image with the second field of view area.

In a first situation, a size of the second field of view area is less than or equal to a size of the second display. In this situation, when the second image with the second field of view area is displayed on the second display, the second display can display a complete second image with the second field of view area (i.e. display the complete circular second image).

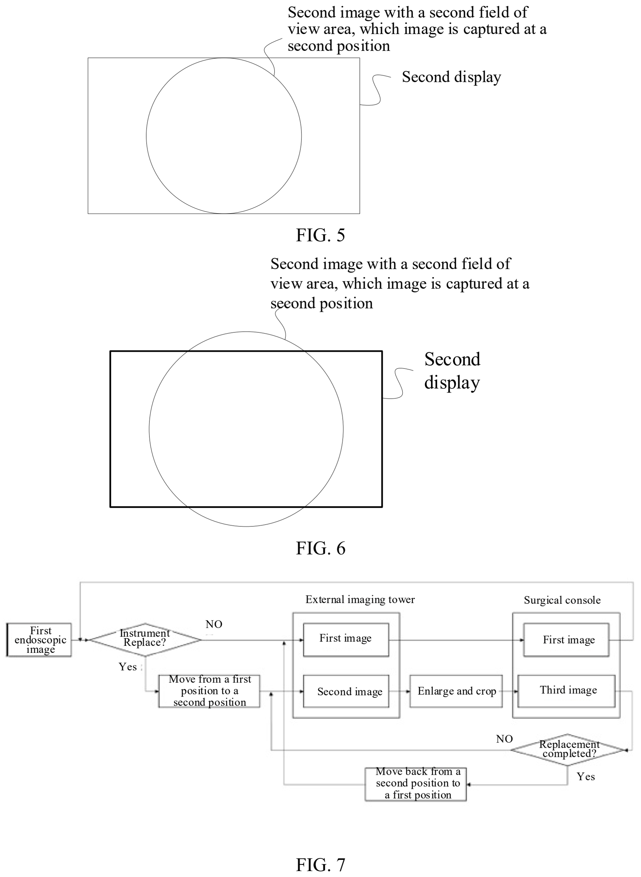

For example, as shown in FIG. 5, which is an example display diagram for a second image with a second field of view area, which image is captured at the second position in an embodiment of this disclosure, when a size of the second field of view area is less than or equal to a size of the second display, the second display can display the complete second image with the second field of view area.

In a second situation, the size of the second field of view area is larger than the size of the second display. In this situation, when the second image with the second field of view area is displayed on the second display, most content of the second image with the second field of view area can be displayed on the second display (i.e., most of the second image is displayed), and the displayed "most content of the second image" corresponds to a field of view area, that is still larger than the first field of view area.

For example, as shown in FIG. 6, which is another example display diagram for a second image with a second field of view area, which image is captured at the second position in an embodiment of this disclosure, the size of the second field of view area is larger than the size of the second display, and most content of the second image with the second field of view area is displayed on the second display.

The second display can expand the field of view area, no matter displaying the complete second image or displaying most content of the second image.

In some specific embodiments, after controlling the endoscope to move to the second position and to capture the second endoscopic image, in order to ensure that a display content of the first display arranged on the surgical console remains unchanged (with the aim of keeping an observation field of view area of a chief surgeon unchanged), maintain the first display to display a third image with the first field of view area based on the second endoscopic image. A content of the third image with the first field of view area includes the content of the first image with the first field of view area.

The implementation method for maintaining the first display to display a third image with the first field of view area based on the second endoscopic image, includes: enlarging and cropping the second endoscopic image to obtain the third image with the first field of view area, and displaying, on the first display, the third image with the first field of view area.

Specifically, based on a distance from the first position to the second position (equivalent to a movement distance of the endoscope) and a working distance of the endoscope at the first position, calculate a magnification ratio (also known as a magnification factor).

For example, the magnification ratio m can be calculated by m=1+sin (60 °) * Y/X, wherein X is the working distance of the endoscope at the first position and Y is the distance from the first position to the second position.

Enlarge the second endoscopic image according to the magnification ratio calculated above, crop a third image with the first field of view area from the enlarged second endoscopic image, and display, on the first display, the third image with the first field of view area.

For example, after enlarging the second endoscopic image by m times (wherein m is the magnification ratio), cropping from a top center area of the enlarged second endoscopic image to produce the third image with the first field of view area, and then display, on the first display, the third image with the first field of view area.

In some specific embodiments, after switching the second display to display a second image with a second field of view area and detecting that the instrument replacement is completed; control the endoscope of the surgical robot system to move back to the first position to recapture the first endoscopic image by the endoscope of the surgical robot system at the first position; and respectively switch back to display, on the first display and the second display, a first image with the first field of view area, based on said first endoscopic image.

Specifically, determine that the instrument replacement is completed, when an operation for characterizing that the instrument replacement is completed, which operation is triggered by a user, is detected; control the endoscope of the surgical robot system to move back to the first position to recapture a first endoscopic image by the endoscope of the surgical robot system at the first position; and respectively switch back to display, on the first display and the second display, a first image with the first field of view area, based on said first endoscopic image.

For example, when an operation for locking an instrument, which operation is triggered by a user, is detected, determine that the instrument replacement is completed. At this time, control the endoscope to move back from the second position to the first position along an axial direction toward an inside of a human body, that is, control the endoscope to move back from the second position to the first position along a central axis of the endoscope in a direction towards an inside of a human body, and recapture a first endoscopic image by the endoscope of the surgical robot system at the first position, and then switch back to display, on the first display and the second display, a first image with the first field of view area, based on said first endoscopic image.

Based on the content shown in each step of FIG. 2 above, one of application situations of this disclosure is as follows. Capture, by an endoscope, a first endoscopic image at a first position. Respectively display a first image with a first field of view area on a first display of a surgical console and a second display of an external image tower, based on the first endoscopic image. When an instrument is replaced, control the endoscope to move along a central axis of the endoscope toward an outside of a human body from the first position to a second position, so as to capture a second endoscopic image. Based on the second endoscopic image, switch the second display to display a second image with a second field of view area, so as to expand an observation field of view area of the external imaging tower, assist an assistant in observing an end position of the instrument to avoid accidental injury. Enlarge and crop the second endoscopic image to obtain a third image with the first field of view area, and maintain displaying the third image with the first field of view area on the first display, so as to remain an observation field of view area of the surgical console unchanged. After the instrument replacement is completed, control the endoscope to move back from the second position to the first position along the central axis of the endoscope toward an inside of the human body. Recapture a first endoscopic image by the endoscope at the first position, and switch the first display and the second display back to display a first image with a first field of view area, based on said first endoscopic image.

To better explain the above application situation, an example workflow diagram for a method for displaying a field of view area of an endoscopic image in an embodiment of this disclosure shown in FIG. 7, is described as follows for example.

Obtain a first endoscopic image, which is captured by an endoscope at a first position. Determine whether to replace an instrument.

If no instrument is replaced, respectively display, on a first display of a surgical console and a second display of an external imaging tower, a first image with a first field of view area, based on the first endoscopic image.

If an instrument is replaced, move the endoscope from the first position to a second position and capture a second endoscopic image by the endoscope at the second position. Based on the second endoscopic image, switch the second display on the external imaging tower to display a second image with a second field of view area; enlarge and crop the second endoscopic image, so as to enable the first display on the surgical console to maintain displaying a third image with the first field of view area, and determine whether the instrument replacement is completed.

If the instrument replacement is completed, move the endoscope back from the second position to the first position and recapture a first endoscopic image by the endoscope at the first position. Based on said first endoscopic image, switch the first display on the surgical console and the second display on the external imaging tower back to display a first image with the first field of view area.

If the instrument replacement is not completed, maintain the first display on the surgical console displaying the third image with the first field of view area, and maintain the second display on the external imaging tower displaying the second image with the second field of view area.

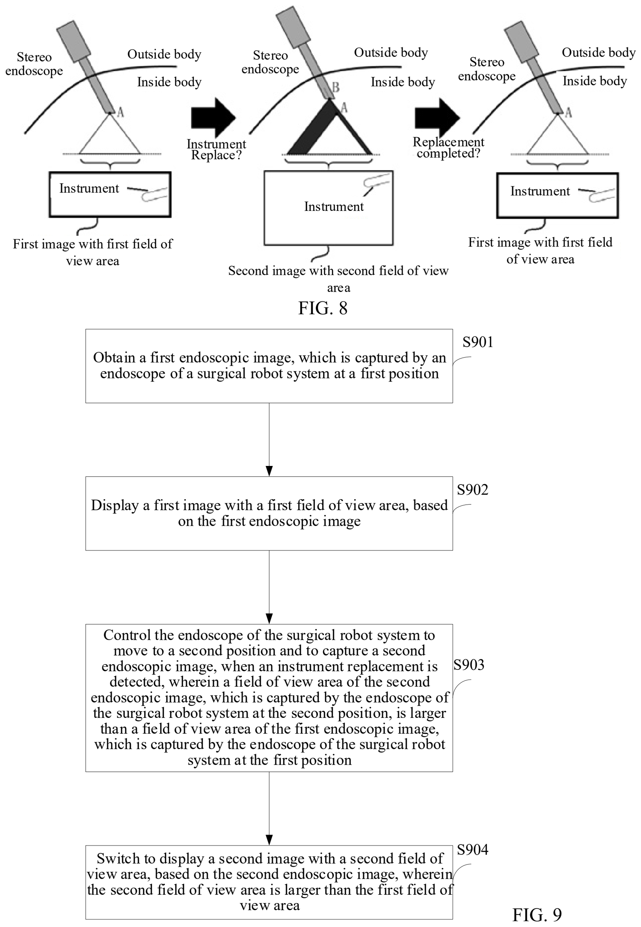

Based on the workflow of the method for displaying a field of view area of an endoscopic image shown in FIG. 7 above, a switching situation for a field of view area of a second display of an external imaging tower is shown in FIG. 8. Point A in FIG. 8 is the first position, point B in FIG. 8 is the second position, and both the first field of view area and second field of view area in FIG. 8 are rectangular. When no instrument is replaced, display the first image with the first field of view area on the second display on the external imaging tower, based on the first endoscopic image captured by the endoscope at the first position. When an instrument is replaced, control the endoscope to move from the first position to the second position and to capture a second endoscopic image with a larger field range of view. Based on the second endoscopic image, switch the second display on the external imaging tower to display a second image with a second field of view area. When the instrument replacement is completed, control the endoscope to move back from the second position to the first position and to recapture a first endoscopic image. Based on said first endoscopic image, switch the second display on the external imaging tower back to display a first image with the first field of view area.

From a switching situation for a field of view area of a second display of an external imaging tower shown in FIG. 8, it can be seen that, when an instrument is replaced, the endoscope is moved, so as to switch the second display on the external imaging tower to display the second image with a larger field of view area, which can identify and observe an instrument outside the first field of view area and avoid accidental injury, during a replacement process of said instrument. Throughout the process, the first display on the surgical console always maintains the same observation field of view area, without affecting or interfering with a display content of the surgical console.

Referring to FIG. 9, a second flowchart for a method for displaying a field of view area of an endoscopic image in an embodiment of this disclosure is shown, which includes following steps.

In step S901, obtain a first endoscopic image, which is captured by an endoscope of the surgical robot system at a first position.

In a specific implementation of step S901, during a surgical process, the first endoscopic image, which is captured by the endoscope (e.g. stereo endoscope) of the surgical robot system (e.g. laparoscopic surgical robot system) at the first position, is obtained.

In step S902, display a first image with a first field of view area, based on the first endoscopic image.

In a specific implementation of step S902, select the first image with the first field of view area from the captured first endoscopic image, and display the first image with the first field of view area.

In step S903, control the endoscope of the surgical robot system to move to a second position and to capture a second endoscopic image, when an instrument replacement is detected, wherein a field of view area of the second endoscopic image, which is captured by the endoscope of the surgical robot system at the second position, is larger than a field of view area of the first endoscopic image, which is captured by the endoscope of the surgical robot system at the first position.

In a specific implementation of step S903, when an instrument replacement is detected, control the endoscope of the surgical robot system to move from the first position to the second position, and to capture a second endoscopic image at the second position.

In some specific embodiments, when an operation for characterizing the instrument replacement, which operation is triggered by a user, is detected, determine that the instrument replacement is detected, and control the endoscope of the surgical robot system to move to the second position, and to capture the second endoscopic image.

In step S904, switch to display a second image with a second field of view area, based on the second endoscopic image, wherein the second field of view area is larger than the first field of view area.

In a specific implementation of step S904, after controlling the endoscope to capture a second endoscopic image at the second position, select the second image with the second field of view area from the second endoscopic image, and switch to display the second image with the second field of view area to expand an observation field of view area.

In some specific embodiments, a content of the second image with the second field of view area includes a content of the first image with the first field of view area.

In other specific embodiments, the first field of view area is rectangular and the second field of view area is circular.

After switching to display a second image with a second field of view area, when detecting that the instrument replacement is completed, control the endoscope of the surgical robot system to move back to the first position and to recapture a first endoscopic image by the endoscope of the surgical robot system at the first position. Specifically, when an operation for characterizing that the instrument replacement is completed, which operation is triggered by a user, is detected, determine that the instrument replacement is completed, and control the endoscope of the surgical robot system to move back to the first position and recapture a first endoscopic image by the endoscope of the surgical robot system at the first position.

After controlling the endoscope to move back from the second position to the first position and to recapture a first endoscopic image by the endoscope of the surgical robot system at the first position, switch back to display a first image with the first field of view area based on said first endoscopic image.

In some specific embodiments, the surgical robot system includes a first display arranged on the surgical console and a second display arranged on the external imaging tower. Therefore, the specific implementation of steps S902 and S904 is as follows: based on the first endoscopic image, respectively display, on the first display and the second display, the first image with the first field of view area; when detecting an instrument replacement, control the endoscope of the surgical robot system to move to the second position, and to capture the second endoscopic image; and just switch the second display to display the second image with the second field of view area, based on the second endoscopic image.

That is to say, based on the first endoscopic image, respectively display, on the first display and the second display, the first image with the first field of view area; when detecting an instrument replacement, control the endoscope to move to the second position and to capture a second endoscopic image; just switch the second display arranged on the external imaging tower to display the second image with the second field of view area based on the second endoscopic image, while maintain an observation field of view area of the first display arranged on the surgical console unchanged. This not only allows an assistant to observe an end position of the instrument through the second image with a larger field of view area, so as to avoid accidental injury, but also ensures that an observation field of view area of a chief surgeon remains unchanged (i.e. does not affect or interfere with a display content of the surgical console).

After controlling the endoscope to move to the second position and to capture the second endoscopic image, in order to maintain the observation field of view area of the first display unchanged, in some specific embodiments, a third image with the first field of view area is displayed on the first display based on the second endoscopic image. Specifically, the second endoscopic image is enlarged and cropped to obtain the third image with the first field of view area, which is then displayed on the first display.

Furthermore, after controlling the endoscope to move to the second position and to capture the second endoscopic image, calculate a magnification ratio, based on a distance from the first position to the second position (equivalent to a movement distance of the endoscope) and a working distance of the endoscope at the first position; enlarge the second endoscopic image according to the magnification ratio, crop a third image with the first field of view area from the enlarged second endoscopic image, and display the third image with the first field of view area on the first display, thereby maintaining the observation field of view area of the first display unchanged.

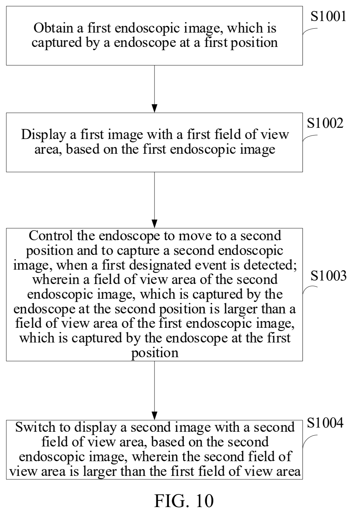

Referring to FIG. 10, a third flowchart for a method for displaying a field of view area of an endoscopic image for a surgical robot system in an embodiment of this disclosure is shown, which includes following steps.

In step S1001, obtain a first endoscopic image, which is captured by a endoscope at a first position.

In a specific implementation of step S1001, during a surgical process, the first endoscopic image, which is captured by the endoscope (such as a stereo endoscope) at the first position, is obtained.

In step S1002, display a first image with a first field of view area, based on the first endoscopic image.

In a specific implementation of step S1002, select the first image with the first field of view area from the captured first endoscopic image, and display the first image with the first field of view area.

In step S1003, control the endoscope to move to the second position and to capture a second endoscopic image, when a first designated event is detected; wherein a field of view area of the second endoscopic image, which is captured by the endoscope at the second position is larger than a field of view area of the first endoscopic image, which is captured by the endoscope at the first position.

In a specific implementation of step S1003, after displaying the first image with the first field of view area, when detecting the first designated event, control the endoscope to move from the first position to the second position to obtain the second endoscope image, which is captured by the endoscope at the second position.

In some specific embodiments, the first designated event is "an instrument replacement" or "an operation for turning on a function for switching a field of view area". After displaying the first image with the first field of view area, control the endoscope to move to the second position, when an instrument replacement is detected, or when an operation for turning on a function for switching a field of view area, which operation is triggered by a user, is detected

For example, a user can turn on a function for switching a field of view area on a surgical console or an external imaging tower. When detecting that the function for switching a field of view area is turned on, control the endoscope to move from the first position to the second position and to obtain the second endoscope image, which is captured by the endoscope at the second position.

In step S1004, switch to display a second image with a second field of view area, based on the second endoscopic image, wherein the second field of view area is larger than the first field of view area.

In a specific implementation of step S1004, after controlling the endoscope to capture a second endoscopic image at the second position, select the second image with the second field of view area from the second endoscopic image, and switch to display the second image with the second field of view area to expand an observation field of view area. A content of the second image with the second field of view area includes a content of the first image with the first field of view area.

In some specific embodiments, after switching to display a second image with a second field of view area, when a second designated event is detected, control the endoscope to move back to the first position and switch back to display the first image with the first field of view area.

Wherein, the second designated event is "completing an instrument replacement" or "an operation for turning off a function for switching a field of view area". After switching to display a second image with a second field of view area, control the endoscope to move back to the first position and switch back to display the first image with the first field of view area, when the instrument replacement is determined to be completed, or when an operation for turning off a function for switching a field of view area, which operation is triggered by a user, is detected.

Specifically, when detecting that the instrument replacement is completed or detecting an operation for turning off a function for switching a field of view area, which operation is triggered by a user, control the endoscope to move back from the second position to the first position and to recapture a first endoscopic image at the first position, and switch back to display a first image with the first field of view area based on said first endoscopic image.

For example, after switching to display a second image with a second field of view area, a user can turn off a function for switching a field of view area on a surgical console or an external imaging tower. When the function for switching a field of view area is detected to be turned off, control the endoscope to move back from the second position to the first position and switch back to display the first image with the first field of view area.

Turning on of functions corresponding to the first designated event and the second designated event can be achieved by triggering physical or virtual key(s), or by automatically detecting certain related action(s).

From the above example, it can be seen that a user can switch to display the second image with the second field of view area by actively turning on the function for switching a field of view area. User can also actively turn off the function for switching a field of view area to switch back to display the first image with the first field of view area, after switching to display a second image with a second field of view area.

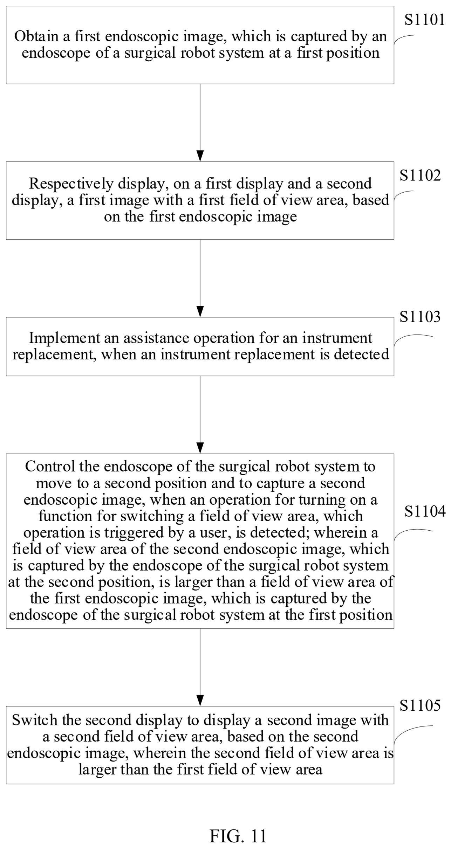

Referring to FIG. 11, a fourth flowchart for a method for displaying a field of view area of an endoscopic image for a surgical robot system in an embodiment of this disclosure is shown. The surgical robot system at least includes a first display arranged on a surgical console and a second display arranged on an external imaging tower. FIG. 11 includes following steps.

In step S1101, obtain a first endoscopic image, which is captured by an endoscope of the surgical robot system at a first position.

In a specific implementation of step S1101, during a surgical process, the first endoscopic image, which is captured by the endoscope (e.g. stereo endoscope) of the surgical robot system (e.g. laparoscopic surgical robot system) at the first position, is obtained.

In step S1102: respectively display, on the first display and the second display, a first image with a first field of view area, based on the first endoscopic image.

In a specific implementation of step S1102, select the first image with the first field of view area from the captured first endoscopic image, and then respectively display, on the first display arranged on the surgical console and the second display arranged on the external imaging tower, the first image with the first field of view area.

In step S1103, implement an assistance operation for an instrument replacement, when an instrument replacement is detected.

In a specific implementation of step S1103, implement the assistance operation for an instrument replacement, so as to assist the instrument replacement, when the instrument replacement is detected.

Specifically, a specific way for the assistance operation for an instrument replacement, is as follows. When detecting that an instrument on the surgical robotic arm is unlocked, an instrument in-position detection is triggered, so as to detect whether said instrument is in position; when said instrument is detected to be out of position (indicating that said instrument has been disassembled), display first prompt information on the first display and the second display to indicate that the instrument has been disassembled. For example, when an instrument is detected to be out of position, display first prompt information "Instrument disassembled" in a bottom status bar of the first display and the second display.

After displaying the first prompt information, when detecting that an instrument is locked on the surgical robotic arm and in position, display second prompt information on the first display and the second display, so as to indicate that said instrument is assembled in position. For example, after displaying the first prompt information, when determining that an instrument is locked to the surgical robotic arm and in position, display second prompt information "Instrument in position" in a bottom status bar of the first display and the second display.

In step S1104, control the endoscope of the surgical robot system to move to a second position and to capture a second endoscopic image, when an operation for turning on a function for switching a field of view area, which operation is triggered by a user, is detected; wherein a field of view area of the second endoscopic image, which is captured by the endoscope of the surgical robot system at the second position, is larger than a field of view area of the first endoscopic image, which is captured by the endoscope of the surgical robot system at the first position.

In a specific implementation of step S1104, after displaying the first image with the first field of view area on the first display and the second display, if an operation for turning on a function for switching a field of view area, which operation is triggered by a user, is detected, control the endoscope of the surgical robot system to move from the first position to the second position, and to capture the second endoscopic image at the second position.

In step S1105, switch the second display to display a second image with a second field of view area, based on the second endoscopic image, wherein the second field of view area is larger than the first field of view area.

In a specific implementation of step S1105, based on the second endoscopic image captured by the endoscope at the second position, switch the second display to display the second image with the second field of view area

Correspondingly, after switching the second display to display the second image with the second field of view area, if an operation for turning off a function for switching a field of view area, which operation is triggered by a user, is detected, control the endoscope to move back to the first position and recapture a first endoscopic image at the first position. Based on said first endoscopic image, switch the second display back to display a first image with the first field of view area.

Combining a process for replacing an instrument in a surgical robot system and applying some embodiments of this disclosure, one practical application situation is as follows. Respectively display a first image with a first field of view area on a first display of a surgical console and a second display of an external imaging tower, based on a first endoscopic image which is captured by an endoscope at a first position.

After determining that an instrument needs to be replaced, an assistant unlocks an instrument to be disassembled on the surgical robotic arm and disassembles said instrument, so as to trigger an instrument in-position detection to detect whether an instrument is in position. When detecting that said instrument is out of position (indicating that said instrument has been disassembled), and detecting an operation for turning on a function for switching a field of view area, which operation is triggered by a user, display first prompt information on the first display and the second display to indicate that the instrument has been disassembled. Control the endoscope to move from a first position to a second position and to capture a second endoscopic image with a larger field of view area. Based on the second endoscopic image, switch the second display on the external imaging tower to display a second image with a second field of view area. Enlarge and crop the second endoscopic image, so as to maintain the first display on the surgical console to display a third image with the first field of view area.

After the assistant completes assembling the new instrument and locks the new instrument again, when detecting that the new instrument is in position and detecting an operation for turning off the function for switching a field of view area, display second prompt information on the first display and the second display to indicate that the new instrument is assembled in position. Control the endoscope to move back from the second position to the first position and recapture a first endoscopic image by the endoscope at the first position. Based on the first endoscopic image, switch back the first display on the surgical console and the second display on the external imaging tower to display the first image with the first field of view area. Throughout the entire process, the first display on the surgical console always maintains the same observation field of view area, without affecting or interfering with a display content of the surgical console.

The above is a partial explanation of the method for displaying a field of view area of an endoscopic image. Through the content of the above embodiments, it is possible to expand the observation field of view area by moving the endoscope during the surgical process.

It is worth noting that, the observation field of view area can also be expanded without moving the endoscope. The following provides a detailed explanation of a method for "expanding an observation field of view without moving an endoscope".

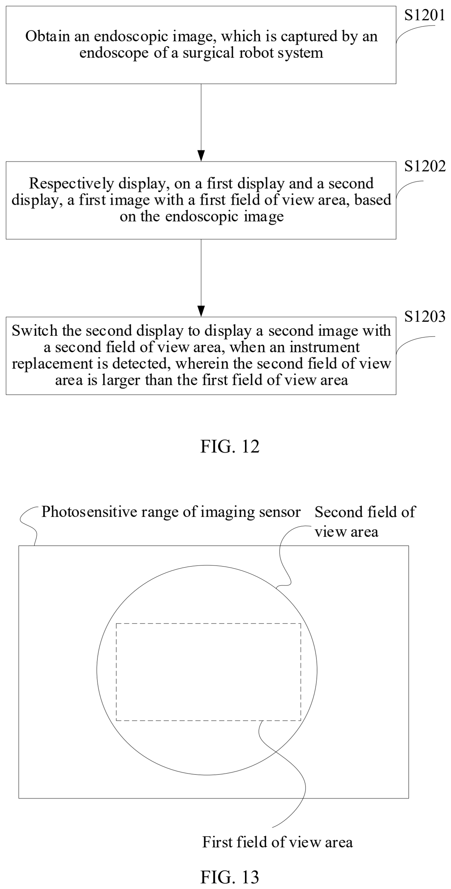

Referring to FIG. 12, a fifth flowchart for a method for displaying a field of view area of an endoscopic image for a surgical robot system in an embodiment of this disclosure is shown. The surgical robot system at least includes a first display arranged on a surgical console and a second display arranged on an external imaging tower. FIG. 12 includes following steps.

In step S1201, obtain an endoscopic image, which is captured by an endoscope of the surgical robot system.

In a specific implementation of step S1201, during a surgical process, an endoscopic image, which is captured by an endoscope (such as a stereo endoscope) of a surgical robot system (such as a laparoscopic surgical robot system), is obtained. Wherein, the endoscope is arranged inside with an image sensor, and the endoscope image is specifically captured by the image sensor.

In step S1202, respectively display, on the first display and the second display, a first image with a first field of view area, based on the endoscopic image.

In a specific implementation of step S1202, select a second image with a second field of view area from the endoscopic image, wherein the second field of view area is smaller than a photosensitive range of the image sensor of the endoscope.

Select the first image with the first field of view area from the second image with the second field of view area, wherein the second field of view area is larger than the first field of view area.

After obtaining the first image with the first field of view area, respectively display the first image with the first field of view area on the first display and the second display.

In some specific embodiments, since the second field of view area is larger than the first field of view area, and the first image with the first field of view area is selected from the second image with the second field of view area, a content of the second image with the second field of view area includes a content of the first image with the first field of view area.

It should be noted that, a shape of a conventional display is usually rectangular. In some specific embodiments, in order to further improve a user experience, the first field of view area can be rectangular (or other shapes), when displaying the first image with the first field of view area on the first display and the second display. This allows the first image with the first field of view area to fill a whole display screen, when displaying the first image with the first field of view area on the first display and the second display.

The image sensor of the endoscope has a field of view area that ensures an optimal imaging quality, and the second field of view area can be said field of view area that ensures an optimal imaging quality, or a partial area within said field of view area that ensures an optimal imaging quality.

Generally speaking, said field of view area that ensures an optimal imaging quality, is usually a circular field of view area, accordingly the second field of view area can be circular (or other shapes).

For example, as shown in FIG. 13, which is an example diagram for a photosensitive range, a first field of view area, and a second field of view area of an image sensor, wherein the second field of view area is 100% of a field of view area that ensures an optimal imaging quality in a photosensitive range of an image sensor (which can be referred to as a 1.0F circular field of view area, wherein 1.0 represents 100%, F represents field); the first field of view area is a rectangular area within the second field of view area. Under a conventional 3D observation, respectively display the first image with the first field of view area on the first display and the second display.

It should be noted that, specific ranges of the photosensitive range, the first field of view area, and the second field of view area of the image sensor shown in FIG. 13, are only for illustration purposes. The first field of view area can also be an inscribed circle within a rectangular photosensitive range of the image sensor, and the second field of view area can also be an inscribed rectangle within a circular first field of view area.

In step S1203, switch the second display to display a second image with a second field of view area, when an instrument replacement is detected, wherein the second field of view area is larger than the first field of view area.

It should be noted that, a process for replacing an instrument in a surgical robot system is as follows. After determining that an instrument needs to be replaced, a user (such as an assistant) unlocks an instrument to be disassembled on the surgical robotic arm, disassembles said instrument, and assembles a new instrument to the surgical robotic arm, and locks the newly assembled instrument.

In a specific implementation of step S1203, when an instrument replacement is detected, switch the second display on the external imaging tower to display the second image with the second field of view area, that is, the second display is switched from displaying the first image with the first field of view area to displaying the second image with the second field of view area, so that the second display expands an observation field of view area.

In some specific embodiments, determine that an instrument replacement is detected, when an operation for characterizing the instrument replacement, which operation is triggered by a user, is detected; and switch the second display to display the second image with the second field of view area.

For example, when an operation for unlocking an instrument, which operation is triggered by a user, is detected, determine that an instrument replacement is detected. At this time, switch the second display to display the second image with the second field of view area, so as to expand an observation field of view area. It is also possible to determine whether a replacement operation for an instrument is implemented by detecting whether the instrument is in position through a robotic arm.

It should be noted that, when the second field of view area is circular, there are two situations, in which the second display is switched to display the second image with the second field of view area.

In a first situation, a size of the second field of view area is less than or equal to a size of the second display. In this situation, when the second image with the second field of view area is displayed on the second display, the second display can display a complete second image with the second field of view area (i.e., display a complete second image).

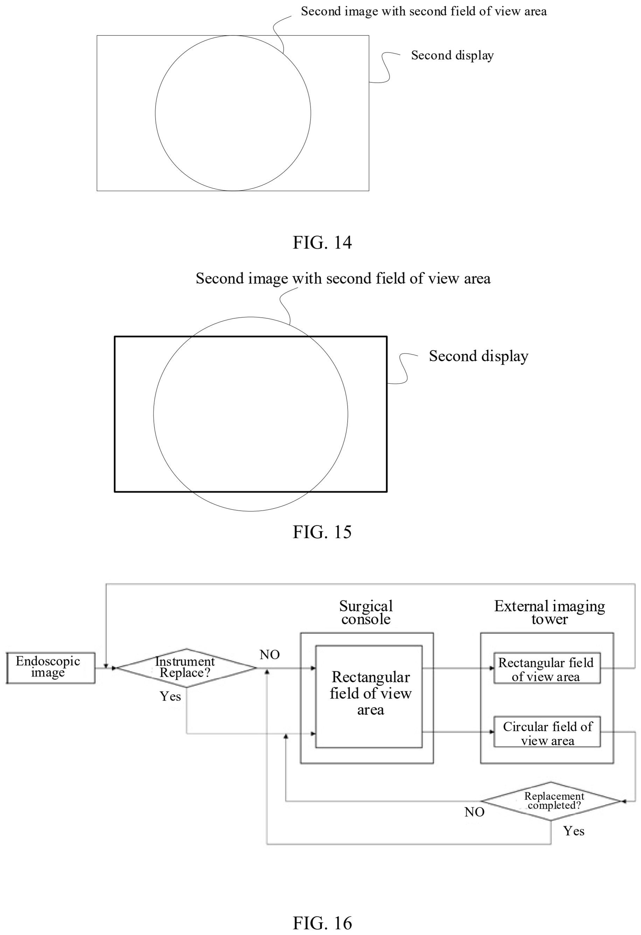

For example, as shown in FIG. 14, which is another example display diagram for a second image with a second field of view area in an embodiment of this disclosure, when the size of the second field of view area is less than or equal to the size of the second display, the second display can display the complete second image with the second field of view area.

In a second situation, the size of the second field of view area is larger than the size of the second display. In this situation, when the second image with the second field of view area is displayed on the second display, most content of the second image with the second field of view area can be displayed on the second display (i.e., most of the second image is displayed), and the displayed "most content of the second image" corresponds to a field of view area, that is still larger than the first field of view area.

For example, as shown in FIG. 15, which is another example display diagram for a second image with a second field of view area in an embodiment of this disclosure, the size of the second field of view area is larger than the size of the second display, and most content of the second image with the second field of view area is displayed on the second display.

The second display can expand the field of view area, no matter displaying the complete second image or displaying most content of the second image.

In other specific embodiments, when the instrument replacement is detected and the second display is switched to display the second image with the second field of view area, maintain the first display to display the first image with the first field of view area.

That is to say, when replacing an instrument, the second display is switched to display the second image with the second field of view area (an observation field of view area of the second display is expanded), while the first display still displays the first image with the first field of view area (an observation field of view area of the first display remains unchanged). This not only allows the assistant to observe an end position of the instrument through the second image with a larger field of view area to avoid accidental injury, but also allows the observation field of view area of the chief surgeon to remain unchanged (i.e. focusing on the first image with the first field of view area).

In some specific embodiments, after switching the second display to display the second image with the second field of view area, when the instrument replacement is determined to be completed, switch the second display back to display the first image with the first field of view area.

Specifically, determine that the instrument replacement is completed, when an operation for characterizing that the instrument replacement is completed, which operation is triggered by a user, is detected; and switch the second display back to display the first image with the first field of view area.

For example, when an operation for locking an instrument, which operation is triggered by a user, is detected, determine that the instrument replacement is completed, and switch the second display back to display the first image with the first field of view area.

It is worth noting that, in practical applications, when replacing an instrument, the first display can also be switched from displaying the first image with the first field of view area to displaying the second image with the second field of view area. After the instrument replacement is completed, the first display is switched back to display the first image with the first field of view area. However, a preferred implementation is to maintain the first display screen displaying the first image with the first field of view area throughout an entire surgical process, so that the observation field of view area of the chief surgeon does not change.

Based on the content shown in each step of FIG. 12 above, one of application situations of this disclosure is to respectively display, on a first display of a surgical console and a second display of an external imaging tower, a first image with a rectangular field of view area (a first field of view area); switch the second display to display a second image with a 1.0F circular field of view area (a second field of view area) to expand an observation field of view area of the external imaging tower, when an instrument is replaced, so as to assist an assistant in observing an end position of the instrument to avoid accidental injury, and maintain the first display to display the first image with the rectangular field of view area (the first field of view area); and switch the second display back to display the first image with the rectangular field of view area (the first field of view area), after the instrument replacement is completed.

To better explain the above application situation, another example workflow diagram for a method for displaying a field of view area of an endoscopic image in an embodiment of this disclosure shown in FIG. 16, is described as follows for example. Obtain an endoscopic image. Determine whether to replace an instrument.

If no instrument is replaced, display, on a first display of a surgical console, a first image with a rectangular field of view area (a first field of view area), and display, on a second display of an external imaging tower, the first image with the rectangular field of view area.

If an instrument is replaced, maintain the first display on the surgical console to display the first image with the rectangular field of view area (that is, maintain an observation field of view area of the surgical console unchanged), while switch the second display on the external imaging tower to display a second image with a 1.0F circular field of view area (a second field of view area), so as to expand an observation field of view area of the external imaging tower.

After the second display on the external imaging tower is switched to display the second image with the 1.0F circular field of view area, determine whether the instrument replacement is complete.

If the instrument replacement is completed, maintain the first display on the surgical console to display the first image with the rectangular field of view area, and switch the second display on the external imaging tower back to display the first image with the rectangular field of view area (the first field of view area).

If the instrument replacement is not completed, maintain the first display on the surgical console to display the first image with the rectangular field of view area, and maintain the second display on the external imaging tower to display the second image with the 1.0F circular field of view area (the second field of view area).

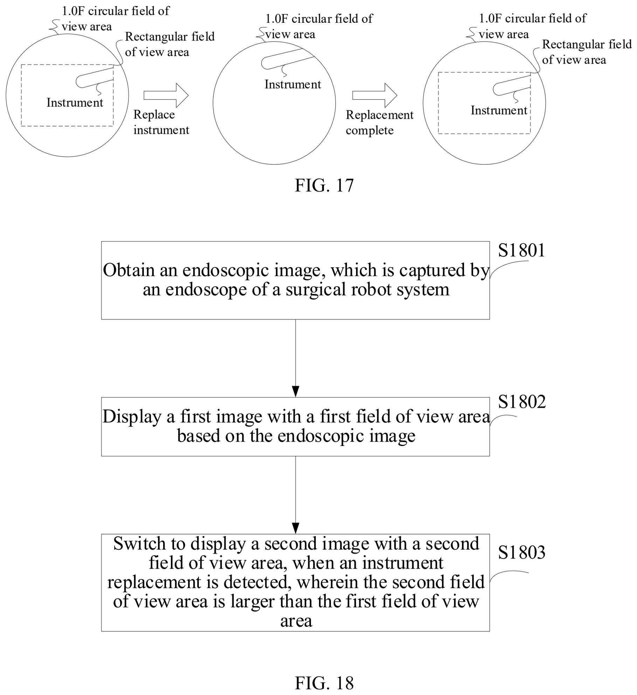

Based on the workflow of the method for displaying a field of view area of an endoscopic image shown in FIG. 16, another switching situation for a field of view area of the second display on the external imaging tower is shown in FIG. 17. In FIG. 17, when no instrument is replaced, the second display on the external imaging tower displays the first image (displayed by a dashed rectangle in FIG. 17) with the rectangular field of view area (the first field of view area); when an instrument is replaced, the second display on the external imaging tower is switched to display the second image of the 1.0F circular field of view area (the second field of view area). When the instrument replacement is completed, the second display is switched back to display the first image (displayed by a dashed rectangle in FIG. 17) with the rectangular field of view area (the first field of view area).

From the switching situation for the field of view area of the second display on the external imaging tower shown in FIG. 17, it can be seen that when an instrument is replaced, the second display on the external imaging tower is switched to display a second image with a larger range for field of view area, which can identify and observe an instrument outside the first field of view area and avoid accidental injury during a replacement process of said instrument.

Combining a process for replacing an instrument in a surgical robot system and applying some embodiments of this disclosure, one practical application situation is as follows. After determining that an instrument needs to be replaced, an assistant unlocks an instrument to be disassembled on the surgical robotic arm and disassembles said instrument. The second display on the external imaging tower is switched to display a second image with a second field of view area, so as to expand a range for a field of view area to assist the assistant in completing the instrument replacement. While the assistant disassembles an instrument and assembles a new instrument, the second display on the external imaging tower continuously displays the second image with the second field of view area. After the assistant completes assembling the new instrument and locks the new instrument again, the second display is switched back to display the first image with the first field of view area. Throughout the entire process, the first display on the surgical console always maintains displaying the first image with the first field of view area, without affecting or interfering with a display content of the surgical console.

Referring to FIG. 18, a sixth flowchart for a method for displaying a field of view area of an endoscopic image in an embodiment of this disclosure is shown, which includes following steps.

In step S1801, obtain an endoscopic image, which is captured by an endoscope of a surgical robot system.

In a specific implementation of step S1801, during a surgical process, an endoscopic image, which is captured by an endoscope (such as a stereo endoscope) of a surgical robot system (such as a laparoscopic surgical robot system), is obtained. Wherein, the endoscope is arranged inside with an image sensor, and the endoscope image is specifically captured by the image sensor.

In step S1802, display a first image with a first field of view area based on the endoscopic image.

In a specific implementation of step S1802, select a second image with a second field of view area from the endoscopic image, wherein the second field of view area is smaller than a photosensitive range of the image sensor of the endoscope.

Select the first image with the first field of view area from the second image with the second field of view area, wherein the second field of view area is larger than the first field of view area.

After obtaining the first image with the first field of view area, display the first image with the first field of view area.

In some specific embodiments, since the second field of view area is larger than the first field of view area, and the first image with the first field of view area is selected from the second image with the second field of view area, a content of the second image with the second field of view area includes a content of the first image with the first field of view area.

In other specific embodiments, the first field of view area may be rectangular (or other shapes), and the second field of view area may be circular (or other shapes).

In step S1803, switch to display a second image with a second field of view area, when an instrument replacement is detected, wherein the second field of view area is larger than the first field of view area.

In a specific implementation of step S1803, when an instrument replacement is detected, switch to display the second image with the second field of view area from the first image with the first field of view area, so as to expand an observation field of view area.

In some specific embodiments, determine that an instrument replacement is detected, when an operation for characterizing the instrument replacement, which operation is triggered by a user, is detected; and switch to display the second image with the second field of view area.

In other specific embodiments, after switching to display a second image with a second field of view area, when the instrument replacement is determined to be completed, switch back to display the first image with the first field of view area.

Specifically, determine that the instrument replacement is completed, when an operation for characterizing that the instrument replacement is completed, which operation is triggered by a user, is detected; and switch back to display the first image with the first field of view area.

In some specific embodiments, the surgical robot system includes a first display arranged on the surgical console and a second display arranged on the external imaging tower. The specific implementation of steps S1802 and S1803 is as follows.

Respectively display, on the first display and the second display, the first image with the first field of view area, based on the endoscopic image; just switch the second display to display the second image with the second field of view area, when an instrument replacement is detected.

That is to say, respectively display, on the first display and the second display, the first image with the first field of view area; when an instrument replacement is detected, just switch the second display arranged on the external imaging tower to display the second image with the second field of view area, and maintain the first display arranged on the surgical console to display the first image with the first field of view area. This not only allows the assistant to observe an end position of the instrument through the second image with a larger field of view area, so as to avoid accidental injury, but also ensures that an observation field of view area of the chief surgeon remains unchanged (i.e. does not affect or interfere with a display content of the surgical console).

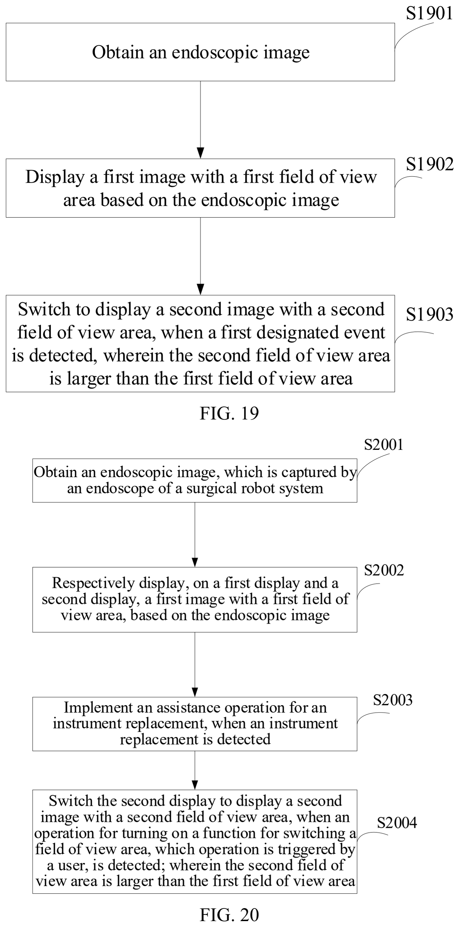

Referring to FIG. 19, a seventh flowchart for a method for displaying a field of view area of an endoscopic image in an embodiment of this disclosure is shown, which includes following steps.

In step S1901, obtain an endoscopic image.

In a specific implementation of step S1901, during a surgical process, an endoscopic image, which is captured by an endoscope (such as a stereo endoscope), is obtained. Wherein, the endoscope is arranged inside with an image sensor, and the endoscope image is specifically captured by the image sensor.

In step S 1902, display a first image with a first field of view area based on the endoscopic image.

In a specific implementation of step S1902, select a second image with a second field of view area from the endoscopic image, wherein the second field of view area is smaller than a photosensitive range of the image sensor of the endoscope.

Select the first image with the first field of view area from the second image with the second field of view area, wherein the second field of view area is larger than the first field of view area.

After obtaining the first image with the first field of view area, display the first image with the first field of view area.

In step S1903, switch to display a second image with a second field of view area, when a first designated event is detected, wherein the second field of view area is larger than the first field of view area.

In a specific implementation of step S1903, when a first designated event is detected, switch from displaying the first image with the first field of view area to displaying the second image with the second field of view area, so as to expand an observation field of view area.

In some specific embodiments, the first designated event is "an instrument replacement" or "an operation for turning on a function for switching a field of view area". After displaying the first image with the first field of view area, switch to display the second image with the second field of view area, when an instrument replacement is detected, or when an operation for turning on a function for switching a field of view area, which operation is triggered by a user, is detected.

For example, a user can turn on the function for switching a field of view area on the surgical console or external imaging tower, so as to switch to display the second image with the second field of view area, that is, a user can actively switch to display the second image with the second field of view area.

In some specific embodiments, after switching to display a second image with a second field of view area, when a second designated event is detected, switch back to display the first image with the first field of view area.

Wherein, the second designated event is "completing an instrument replacement" or "an operation for turning off a function for switching a field of view area". After switching to display a second image with a second field of view area, switch back to display the first image with the first field of view area, when the instrument replacement is determined to be completed, or when an operation for turning off a function for switching a field of view area, which operation is triggered by a user, is detected.

For example, after switching to display a second image with a second field of view area, a user can turn off a function for switching a field of view area on the surgical console or external imaging tower, so as to switch back to display the first image with the first field of view area. That is, the user can actively switch back to display the first image with the first field of view area.

Turning on of functions corresponding to the first designated event and the second designated event can be achieved by triggering physical or virtual key(s), or by automatically detecting certain related action(s).

Referring to FIG. 20, an eighth flowchart for a method for displaying a field of view area of an endoscopic image for a surgical robot system in an embodiment of this disclosure is shown. The surgical robot system at least includes a first display arranged on a surgical console and a second display arranged on an external imaging tower. FIG. 20 includes following steps.

In step S2001, obtain an endoscopic image, which is captured by an endoscope of the surgical robot system.

In a specific implementation of step S2001, during a surgical process, an endoscopic image, which is captured by an endoscope (such as a stereo endoscope) of a surgical robot system (such as a laparoscopic surgical robot system), is obtained. Wherein, the endoscope is arranged inside with an image sensor, and the endoscope image is specifically captured by the image sensor.

In step S2002, respectively display, on the first display and the second display, a first image with a first field of view area, based on the endoscopic image.

In a specific implementation of step S2002, select a second image with a second field of view area from the endoscopic image, wherein the second field of view area is smaller than a photosensitive range of the image sensor of the endoscope.

Select the first image with the first field of view area from the second image with the second field of view area, wherein the second field of view area is larger than the first field of view area.

After obtaining the first image with the first field of view area, respectively display the first image with the first field of view area on the first display and the second display.

In step S2003, implement an assistance operation for an instrument replacement, when an instrument replacement is detected.

In a specific implementation of step S2003, implement the assistance operation for an instrument replacement, so as to assist the instrument replacement, when the instrument replacement is detected.

Specifically, a specific way for the assistance operation for an instrument replacement, is as follows. When detecting that an instrument on the surgical robotic arm is unlocked, an instrument in-position detection is triggered, so as to detect whether said instrument is in position; when said instrument is detected to be out of position (indicating that said instrument has been disassembled), display first prompt information on the first display and the second display to indicate that the instrument has been disassembled. For example, when an instrument is detected to be out of position, display first prompt information "Instrument disassembled" in a bottom status bar of the first display and the second display.

After displaying the first prompt information, when detecting that an instrument is locked on the surgical robotic arm and in position, display second prompt information on the first display and the second display, so as to indicate that said instrument is assembled in position. For example, after displaying the first prompt information, when determine that an instrument is locked to the surgical robotic arm and in position, display second prompt information "Instrument in position" in a bottom status bar of the first display and the second display.

In step S2004, switch the second display to display a second image with a second field of view area, when an operation for turning on a function for switching a field of view area, which operation is triggered by a user, is detected; wherein the second field of view area is larger than the first field of view area.

In a specific implementation of step S2004, after displaying the first image with the first field of view area on the first display and the second display, if an operation for turning on a function for switching a field of view area, which operation is triggered by a user, is detected, switch the second display to display the second image with the second field of view area. That is, the user can actively switch to display the second image with the second field of view area, so as to expand an observation field of view area.

Correspondingly, after switching the second display to display the second image with the second field of view area; when an operation for turning off a function for switching a field of view area, which operation is triggered by a user, is detected, switch the second display back to display the first image with the first field of view area.

Combining a process for replacing an instrument in a surgical robot system and applying some embodiments of this disclosure, one practical application situation is as follows. After determining that an instrument needs to be replaced, an assistant unlocks an instrument to be disassembled on the surgical robotic arm and disassembles said instrument, so as to trigger an instrument in-position detection to detect whether an instrument is in position. When detecting that said instrument is out of position (indicating that said instrument has been disassembled), and detecting an operation for turning on a function for switching a field of view area, which is triggered by a user, display first prompt information on the first display and the second display to indicate that the instrument has been disassembled, and switch the second display on the external imaging tower to display a second image with a second field of view area, so as to expand a range for a field of view area to assist the assistant in completing the instrument replacement.

While the assistant disassembles an instrument and assembles a new instrument, the second display continuously displays a second image with a second field of view area. After the assistant completes assembling the new instrument and locks the new instrument again, when detecting that the new instrument is in position and an operation for turning off the function for switching a field of view area, display second prompt information on the first display and the second display to indicate that the new instrument is assembled in position, and switch the second display back to display the first image with the first field of view area. Throughout the entire process, the first display on the surgical console always maintains displaying the first image with the first field of view area, without affecting or interfering with a display content of the surgical console.

The above is a partial explanation of the method for displaying a field of view area of an endoscopic image. Through the content of the above embodiments, it is possible to expand an observation field of view area during a surgical process.

In summary, this disclosure proposes a method for displaying a field of view area of an endoscopic image and a surgical robot system. When a surgical robot system is detected to replace an instrument, move an endoscope to obtain an endoscopic image with a larger field of view area, and switch to display an image with a larger field of view area based on the endoscopic image with a larger field of view area, which image is obtained by imaging after the movement, so as to assist a user to observe an end position of the instrument in real time, thus avoiding accidental contact between the instrument and human body, reducing a risk of accidental injury and improving a user experience.

The various embodiments in this description are described in a progressive manner, and the same and similar parts between each embodiment can be referred to each other. Each embodiment focuses on differences from other embodiments. Especially for systems or system embodiments, due to their basic similarity to method embodiments, the description is relatively simple. For relevant information, please refer to the section on method embodiments. The system and system embodiments described above are only illustrative, where the units described as separate components may or may not be physically separated, and the components displayed as units may or may not be physical units, i.e. they may be located in one place or distributed across multiple network units. Some or all of the modules can be selected according to actual needs to achieve the purpose of this embodiment. Ordinary technical personnel in this field can understand and implement without putting in creative labor.

Professionals can further realize that the units and algorithm steps described in the examples disclosed in this disclosure can be implemented using electronic hardware, computer software, or a combination of both. In order to clearly illustrate the interchangeability of hardware and software, the composition and steps of each example have been generally described in the above description according to their functions. Whether these functions are executed in hardware or software depends on the specific application and design constraints of the technical solution. Professional technicians can use different methods to achieve the described functions for each specific application, but such implementation should not be considered beyond the scope of this disclosure.

The above description of the disclosed embodiments enables those skilled in the art to implement or use this disclosure. Various modifications to these embodiments will be apparent to those skilled in the art, and the general principles defined herein can be implemented in other embodiments without departing from the scope of this disclosure. Therefore, this disclosure will not be limited to the embodiments shown herein, but will be within the widest scope consistent with the principles and novel features disclosed herein.

## Claims

1. A method for displaying a field of view area of an endoscopic image for a surgical robot system, wherein the surgical robot system at least comprises a first display arranged on a surgical console and a second display arranged on an external imaging tower;
**characterized in that**, the method comprises:
obtaining a first endoscopic image, which is captured by an endoscope of the surgical robot system at a first position;
respectively displaying, on the first display and the second display, a first image with a first field of view area, based on the first endoscopic image;
controlling the endoscope of the surgical robot system to move to a second position and to capture a second endoscopic image, when an instrument replacement is detected, wherein a field of view area of the second endoscopic image, which is captured by the endoscope of the surgical robot system at the second position, is larger than a field of view area of the first endoscopic image, which is captured by the endoscope of the surgical robot system at the first position; and
switching the second display to display a second image with a second field of view area, based on the second endoscopic image, wherein the second field of view area is larger than the first field of view area.

2. The method according to claim 1, **characterized in that**, further comprising:
maintaining the first display to display a third image with the first field of view area, based on the second endoscopic image, when the instrument replacement is detected, while controlling the endoscope of the surgical robot system to move to the second position and to capture the second endoscopic image.

3. The method according to claim 2, **characterized in that**, maintaining the first display to display a third image with the first field of view area, based on the second endoscopic image, comprises:
enlarging and cropping the second endoscopic image to obtain the third image with the first field of view area; and
displaying, on the first display, the third image with the first field of view area.

4. The method according to any one of claims 1-3, **characterized in that**, controlling the endoscope of the surgical robot system to move to a second position and to capture a second endoscopic image, when an instrument replacement is detected, comprises:
determining that the instrument replacement is detected, when an operation for characterizing the instrument replacement, which operation is triggered by a user, is detected; and
controlling the endoscope of the surgical robot system to move to the second position and to capture the second endoscopic image.

5. The method according to any one of claims 1-4, **characterized in that**, further comprising:
controlling the endoscope of the surgical robot system to move back to the first position and to recapture a first endoscopic image; when the instrument replacement is determined to be completed; and
switching back to respectively display, on the first display and the second display, a first image with the first field of view area, based on said first endoscopic image.

6. The method according to claim 5, **characterized in that**, controlling the endoscope of the surgical robot system to move back to the first position and to recapture a first endoscopic image; when the instrument replacement is determined to be completed; comprises:
determining that the instrument replacement is completed, when an operation for characterizing that the instrument replacement is completed, which operation is triggered by a user, is detected; and
controlling the endoscope of the surgical robot system to move back to the first position and to recapture the first endoscopic image.

7. The method according to any one of claims 1-6, **characterized in that**, a content of the second image with the second field of view area comprises a content of the first image with the first field of view area.

8. The method according to any one of claims 1-7, **characterized in that**, the first field of view area is rectangular, and the second field of view area is circular.

9. A method for displaying a field of view area of an endoscopic image, **characterized in that**, comprising:
obtaining a first endoscopic image, which is captured by an endoscope of a surgical robot system at a first position;
displaying a first image with a first field of view area, based on the first endoscopic image;
controlling the endoscope of the surgical robot system to move to a second position and to capture a second endoscopic image, when an instrument replacement is detected, wherein a field of view area of the second endoscopic image, which is captured by the endoscope of the surgical robot system at the second position, is larger than a field of view area of the first endoscopic image, which is captured by the endoscope of the surgical robot system at the first position; and
switching to display a second image with a second field of view area, based on the second endoscopic image, wherein the second field of view area is larger than the first field of view area.

10. A method for displaying a field of view area of an endoscopic image, **characterized in that**, comprising:
obtaining a first endoscopic image, which is captured by an endoscope at a first position;
displaying a first image with a first field of view area, based on the first endoscopic image;
controlling the endoscope to move to a second position and to capture a second endoscopic image, when a first designated event is detected, wherein a field of view area of the second endoscopic image, which is captured by the endoscope at the second position, is larger than a field of view area of the first endoscopic image, which is captured by the endoscope at the first position; and
switching to display a second image with a second field of view area, based on the second endoscopic image, wherein the second field of view area is larger than the first field of view area.

11. The method according to claim 10, **characterized in that**, controlling the endoscope to move to a second position, when a first designated event is detected, comprises:
controlling the endoscope to move to the second position, when an instrument replacement is detected, or when an operation for turning on a function for switching a field of view area, which operation is triggered by a user, is detected.

12. The method according to claim 11 or claim 10, **characterized in that**, after switching to display a second image with a second field of view area, the method further comprises:
controlling the endoscope to move back to the first position, and switching back to display the first image with the first field of view area, when a second designated event is detected.

13. The method according to claim 12, **characterized in that**, controlling the endoscope to move back to the first position, and switching back to display the first image with the first field of view area, when a second designated event is detected, comprise:
controlling the endoscope to move back to the first position, and switching back to display the first image with the first field of view area, when an instrument replacement is determined to be completed, or when an operation for turning off a function for switching a field of view area, which operation is triggered by a user, is detected.

14. A method for displaying a field of view area of an endoscopic image for a surgical robot system, wherein the surgical robot system at least comprises a first display arranged on a surgical console and a second display arranged on an external imaging tower;
**characterized in that**, the method comprises:
obtaining a first endoscopic image, which is captured by an endoscope of the surgical robot system at a first position;
respectively displaying, on the first display and the second display, a first image with a first field of view area, based on the first endoscopic image;
implementing an assistance operation for an instrument replacement, when the instrument replacement is detected;
controlling the endoscope of the surgical robot system to move to a second position and to capture a second endoscopic image, when an operation for turning on a function for switching a field of view area, which operation is triggered by a user, is detected; wherein a field of view area of the second endoscopic image, which is captured by the endoscope of the surgical robot system at the second position, is larger than a field of view area of the first endoscopic image, which is captured by the endoscope of the surgical robot system at the first position; and
switching the second display to display a second image with a second field of view area, based on the second endoscopic image, wherein the second field of view area is larger than the first field of view area.

15. A surgical robot system comprising a processor and a memory, **characterized in that**, the memory comprises computer-readable instruction(s) stored therein, wherein the computer-readable instruction(s), when executed by the processor, implements(implement) the method for displaying a field of view area of an endoscopic image according to any one of claims 1-14.
